(19) Europäisches Patentamt / European Patent Office / Office européen des brevets

(11) **EP 0 971 212 B1**

(12) **EUROPÄISCHE PATENTSCHRIFT**

(45) Veröffentlichungstag und Bekanntmachung des Hinweises auf die Patenterteilung:
**20.04.2011 Patentblatt 2011/16**

(51) Int Cl.:
*G01F 1/80* (2006.01)    *G01N 11/16* (2006.01)
*F04D 29/04* (2006.01)    *A61M 1/10* (2006.01)

(21) Anmeldenummer: **99810573.8**

(22) Anmeldetag: **02.07.1999**

(54) **Verfahren zur Bestimmung des Druckverlustes und des Durchflusses durch eine Pumpe**

Method to determine the pressure loss and the flow rate through a pump

Méthode pour déterminer la perte de pression et le débit d'une pompe

(84) Benannte Vertragsstaaten:
**CH DE GB LI**

(30) Priorität: **10.07.1998 EP 98810657**

(43) Veröffentlichungstag der Anmeldung:
**12.01.2000 Patentblatt 2000/02**

(73) Patentinhaber: **Levitronix LLC**
**Waltham, MA 02451 (US)**

(72) Erfinder:
• **Schöb, Reto, Dr.**
 **8604 Volketswil (CH)**
• **Barletta, Natale**
 **8037 Zürich (CH)**
• **Hahn, Jürgen**
 **8003 Zürich (CH)**

(74) Vertreter: **Sulzer Management AG**
**Patentabteilung / 0067**
**Zürcherstrasse 14**
**8401 Winterthur (CH)**

(56) Entgegenhaltungen:
US-A- 4 341 111    US-A- 4 779 614
US-A- 4 781 525    US-A- 5 613 831
US-A- 5 725 357

# EP 0 971 212 B1

## Beschreibung

Die Erfindung betrifft ein Verfahren zur Bestimmung des Drucks und/oder des Durchflusses einer Flüssigkeit in bzw. durch eine Zentrifugalpumpe oder Diagonalpumpe oder Axialpumpe, welche mindestens ein Magnetlager zur axialen Lagerung des Pumpenrotors aufweist, gemäss dem Oberbegriff des unabhängigen Patentanspruchs.

Pumpen, bei denen der Pumpenrotor magnetisch gelagert ist, kommen heutzutage bereits in etlichen Anwendungsgebieten zum Einsatz. Insbesondere - aber nicht ausschliesslich - handelt es sich dabei um solche Anwendungen, bei denen mechanische Lagerungen des Rotors aufgrund von möglicher Verschmutzung des zu fördernden Mediums nicht toleriert werden können, wie dies beispielsweise bei Blutpumpen der Fall ist. Hier sind magnetische Lagerungen des Rotors die Regel.

Pumpen mit magnetischen Lagerungen des Rotors sind beispielsweise aus der DE-A-196 13 388 ($\cong$ US-A-5,735,357), US-A-US 5,613,831, US-A-US 4,779,614, US-A-US 4,341,111 oder aus der US-A-4,781,525 bekannt. Pumpen kann man durch einige charakteristische Grössen näher beschreiben. Zwei wichtige socher charakteristischer Grössen sind der Druck in einer Pumpe und der Durchfluss durch eine Pumpe. Diese Grössen kann man grundsätzlich mit entsprechenden Einrichtungen zur Druckmessung und zur Durchflussmessung bestimmen, jedoch hat dies typischerweise zur Folge, dass entsprechende Sensoren in der Pumpe vorgesehen sein müssen. Gerade bei Blutpumpen ist es jedoch wünschenswert, wenn diese Grössen sensorfrei bestimmt werden können.

Hierzu wird in der bereits genannten US-A-4,781,525 vorgeschlagen, den Durchfluss derart zu bestimmen, dass man bei konstanter Viskosität der zu fördernden Flüssigkeit und bei vorgegebener Rotordrehzahl das Drehmoment bestimmt (über den Strom, mit dem der Rotor angetrieben wird), mit welchem der Rotor angetrieben wird. Dieses Drehmoment hängt gemäss der genannten US-A-4,781,525 (Spalte 1, Zeilen 57 ff., Spalte 3, Zeilen 50 ff) bei konstanter Viskosität mit dem Durchfluss linear zusammen. Der Durchfluss wird mit Hilfe von verschiedenen Tabellen ermittelt (siehe Fig. 2 der genannten US-A-4,781,525), von denen jede für eine konstante Viskosität den linearen Zusammenhang zwischen Drehmoment und Durchfluss repräsentiert. Hierzu ist anzumekren, dass dieser lineare Zusammenhang erstens nur sehr näherungsweise gilt und zweitens nur für begrenzte Durchflussmengen gültig ist. Realistisch betrachtet ist der Zusammenhang zwischen Drehmoment und Durchfluss (bei einer vorgegebenen Drehzahl) jedoch nicht-linear, erst recht gilt dies oberhalb einer bestimmten Durchflussmenge.

Gleichwohl besteht das Bedürfnis, den Druck in einer und/oder den Durchfluss durch eine solche Pumpe sensorfrei (also ohne spezielle Sensoren für Druck und Durchfluss) bestimmen zu können und dabei möglichst realistische Werte für Druck und Durchfluss zu erhalten, ohne den Druck und den Durchfluss direkt messen zu müssen. Dieser Aufgabe widmet sich die vorliegende Erfindung.

Erfindungsgemäss wird ein Verfahren bei dem die verwendete Pumpe mindestens ein Magnetlager zur axialen Lagerung des Pumpenrotors aufweist, wobei beim Betrieb der Pumpe auf den Rotor Axialkräfte wirken. Zur Bestimmung des Drucks und/oder des Durchflusses werden bei dem erfindungsgemässen Verfahren die auf den Rotor wirkenden Axialkräfte herangezogen. Die auf den Rotor wirkenden Axialkräfte lassen sich auf verschiedene Arten einfach bestimmen.

Die auf den Pumpenrotor wirkenden Axialkräfte (Axialschub) sind nämlich abhängig von der Druckdifferenz zwischen der Einlassseite der Pumpe (Zentrifugalpumpe, Diagonalpumpe, Axialpumpe) und ihrer Auslassseite. Daher kann man die auf den Pumpenrotor wirkenden Axialkräfte (Axialschub) dazu nutzen, um diese Druckdifferenz - diese Druckdifferenz wird allgemein als "Druck" in der Pumpe bezeichnet - zu bestimmen.

Bei einer Verfahrensvariante ist die axiale magnetische Lagerung des Pumpenrotors als aktive Lagerung ausgebildet. Zur Ermittlung der auf den Rotor wirkenden Axialkräfte wird dann der zur axialen Lagerung des Rotors jeweils erforderliche Lagerstrom herangezogen.

Bei einer anderen Verfahrensvariante ist die axiale magnetische Lagerung des Pumpenrotors als passive Lagerung ausgebildet. Zur Ermittlung der auf den Rotor wirkenden Axialkräfte wird dann die axiale Auslenkung des Rotors herangezogen.

Je nach Art der axialen magnetischen Lagerung (aktiv bzw. passiv) können verschiedene zur axialen Lagerung des Rotors erforderliche Lagerströme bzw. verschiedene axiale Auslenkungen des Rotors jeweils bei verschiedenen Drehzahlen sowie die jeweiligen daraus resultierenden Drücke in einer elektronischen Nachschlagetabelle abgespeichert sein. Diese Nachschlagetabelle wird vor dem praktischen Einsatz der Pumpe erstellt. Beim Betrieb der Pumpe werden dann der jeweilige Lagerstrom bzw. die jeweilige axiale Auslenkung des Rotors sowie seine Drehzahl ermittelt, und mit Hilfe dieser Nachschlagetabelle wird dann der daraus resultierende Druck bestimmt.

Da von einem Pumpentyp regelmässig eine grössere Stückzahl gefertigt wird, muss die Nachschlagetabelle nur ein einziges Mal erstellt werden, sofern sichergestellt ist, dass die gefertigten Pumpenteile innerhalb vorgegebener Toleranzen bleiben, was aber ohne weiteres möglich ist. Selbstverständlich ist es auch möglich, eine solche Nachschlagetabelle auch für jede einzelne Pumpe zu erstellen und abzuspeichern, wodurch allerdings der Aufwand erhöht wird, dafür aber auch die Genauigkeit verbessert wird.

Als Alternative zu einer Nachschlagetabelle kann - je nach Art der axialen Lagerung (aktiv oder passiv) - der Druck in der Pumpe aus dem jeweiligen zur axialen Lagerung des Rotors erforderlichen Lagerstrom bzw. aus der jeweiligen axialen Auslenkung des Rotors einerseits sowie aus der jeweiligen Drehzahl andererseits mit Hilfe eines Polynoms bestimmt werden. Ein solches Polynom gestattet es ebenfalls, den Druck in der Pumpe mit einer genügenden Genauigkeit zu bestimmen.

Je höher dabei die Genauigkeitsanforderungen sind, desto höher wird auch die Ordnung des Polynoms und desto grösser der Rechenaufwand. Die Koeffizienten des Polynoms werden dabei ebenfalls vor dem praktischen Einsatz der Pumpe ermittelt. Auch hier gilt, dass die Koeffizienten für einen bestimmten Pumpentyp bei Einhaltung vorgegebener Fertigungstoleranzen nur ein einziges Mal ermittelt werden müssen, sie können aber auch für jede Pumpe einzeln ermittelt werden, was die Genauigkeit erhöht, aber auch den Aufwand vergrössert.

Bei einer Weiterbildung der vorgenannten Verfahrensvarianten wird aus dem bereits bestimmten Druck und der zugehörigen Drehzahl der Durchfluss durch die Pumpe bestimmt. Die Bestimmung des Durchflusses erfolgt über die Druck-Durchfluss-Kennlinien der Pumpe (auch "Drosselkurven" genannt).

Diese Druck-Durchfluss-Kennlinien der Pumpe können wieder in einer elektronischen Nachschlagetabelle abgespeichert sein, welche vor dem praktischen Einsatz der Pumpe entweder einmal für jeden Pumepntyp oder für jede Pumpe speziell erstellt worden ist. Beim Betrieb der Pumpe wird dann aus dem bereits bestimmten Druck mit Hilfe dieser Nachschlagetabelle der Durchfluss durch die Pumpe bestimmt.

Alternativ zur Nachschlagetabelle können die Druck-Durchfluss-Kennlinien durch ein Polynom approximiert werden, sodass beim Betrieb der Pumpe aus dem bereits bestimmten Druck mit Hilfe dieses Polynoms dann der Durchfluss durch die Pumpe bestimmt wird. Für die Erstellung des Poylnoms gelten die obigen Betrachtungen.

Bei einer Variante der vorgenannten Verfahren wird eine Pumpe mit Sensoren zur Bestimmung der Richtung des Magnetfelds im Spalt zwischen dem Rotor und dem Stator verwendet. Diese Sensoren (typischerweise Hall-Sensoren) werden bei dieser Verfahrensvariante auch zur Bestimmung der axialen Auslenkung des Rotors verwendet.

Bei dieser Verfahrensvariante kann der Durchfluss durch die Pumpe bestimmt werden, indem das den Rotor antreibende Drehmoment und die Drehzahl des Rotors ermittelt werden. Zur Ermittlung des Drehmoments werden einerseits der magnetische Antriebsfluss im Spalt zwischen Rotor und Stator sowie der Antriebsstrom für den Rotor ermittelt und andererseits wird die axiale Auslenkung des Rotors ermittelt. Aus dem Antriebsstrom und der axialen Auslenkung des Rotors wird dann das antreibende Drehmoment ermittelt, sodass schliesslich aus dem so ermittelten Drehmoment und der ermittelten Drehzahl der Durchfluss durch die Pumpe bestimmt wird.

Hierzu können verschiedene Antriebsströme bei verschiedenen axialen Auslenkungen des Rotors sowie das jeweils daraus resultierende Drehmoment in einer elektronischen Nachschlagetabelle abgespeichert sein, die vor dem praktischen Einsatz der Pumpe erstellt und abgespeichert worden ist. Beim Betrieb der Pumpe wird dann aus der jeweiligen axialen Auslenkung des Rotors und dem jeweiligen Antriebsstrom zunächst mit Hilfe dieser Nachschlagetabelle das Drehmoment ermittelt. Anschliesend wird aus dem so ermittelten Drehmoment und der ermittelten Drehzahl dann der Durchfluss durch die Pumpe bestimmt.

Diese Bestimmung des Durchflusses der Pumpe aus dem ermittelten Drehmoment und der Drehzahl des Pumpenrotors erfolgt über die sogenannten Leistungs-Fluss-Kennlinien. Diese Leistung-Fluss-Kennlinien sind grundsätzlich nicht-linear (im Unterschied zu dem linearen Ansatz für den Zusammenhang zwischen Fluss und Drehmoment in der weiter oben genannten US-A-4,781,525) und können in Form einer elektronischen Nachschlagetabelle abgespeichert sein, sie können aber durch ein Polynom approximiert werden.

Bei einer Weiterbildung der vorstehend genannten Verfahrensvariante wird aus dem bereits bestimmten Durchfluss und der zugehörigen Drehzahl über die Druck-Durchfluss-Kennlinien ("Drosselkurven") der Pumpe der Druck in der Pumpe bestimmt.

Diese Druck-Durchfluss-Kennlinien ("Drosselkurven") der Pumpe können, wie weiter oben bereits erwähnt, in einer elektronischen Nachschlagetabelle abgespeichert sein, sodass beim Betrieb der Pumpe aus dem bereits bestimmten Durchfluss dann mit Hilfe dieser Nachschlagetabelle der Druck in der Pumpe bestimmt wird.

Alternativ können, wie ebenfalls weiter oben schon erwähnt, die Druck-Durchfluss-Kennlinien ("Drosselkurven" )durch ein Polynom approximiert werden, sodass beim Betrieb der Pumpe aus dem bereits bestimmten Durchfluss mit Hilfe dieses Polynoms der Druck in der Pumpe bestimmt wird.

Diejenigen vorstehend beschriebenen Verfahrensvarianten, bei denen der Durchfluss ermittelt wird, sind von einer gegebenen Viskosität ausgegangen. Will man die Genauigkeit nun weiter erhöhen, so kann zum Zweck der Bestimmung des Durchflusses aus dem zuvor bestimmten Druck bzw. zum Zweck der Bestimmung des Durchflusses aus dem Drehmoment und der Drehzahl die Viskosität der Flüssigkeit in der Pumpe ermittelt werden. Hierzu bieten sich folgende Varianten an.

In einer ersten Variante wird zur Ermittlung der Viskosität der Flüssigkeit der mit Hilfe der auf den Rotor wirkenden Axialkräfte bestimmte Druck verglichen mit dem Druck, der sich aus der Bestimmung mit Hilfe des ermittelten Drehmoments und der ermittelten Drehzahl ergibt. Dazu muss der Druck allerdings auch auf beide Arten bestimmt werden. Aus der Differenz dieser beiden Drücke und aus der zugehörigen Drehzahl wird dann die Viskosität bestimmt.

Bei einer zweiten Variante wird zur Bestimmung der Viskosität der Flüssigkeit die auf den Rotor wirkende Flüssigkeitsdämpfung ermittelt und aus der ermittelten Flüssigkeitsdämpfung wird dann die Viskosität bestimmt. Das ist deshalb möglich, weil jedes Magnetlager durch seine Steifigkeit und durch seine Dämpfung charakterisiert werden kann. Physikalisch betrachtet ist die Viskosität ein Mass für die (geschwindigkeitsproportionale) Flüssigkeitsreibung und hat somit die Wirkung einer Dämpfung.

Befindet sich nun der magnetgelagerte Rotor in einer Flüssigkeit, so wird im Vergleich zum Betrieb in Luft vor allen Dingen die Gesamtdämpfung des Systems vergrössert. Da sich die Gesamtdämpfung des Systems im wesentlichen aus der Dämpfung des Magnetlagers und der Flüssigkeitsdämpfung additiv zusammensetzt, bildet die Flüssigkeitsdämpfung ein Mass für die Viskosität der Flüssigkeit. Die Flüssigkeitsdämpfung kann nun entweder direkt ermittelt werden oder indirekt über die Systemdämpfung (die sich ja aus der bekannten Dämpfung des Magnetlagers und der Flüssigkeitsdämpfung additiv zusammensetzt). Hierzu bieten sich mehrere Varianten an.

In einer ersten Variante wird die Flüssigkeitsdämpfung mit Hilfe der Verschiebung der Eigenfrequenzen (Starrkörperschwingungen, Biegeschwingungen) des Rotors ermittelt.

In einer zweiten Variante wird die Flüssigkeitsdämpfung ermittelt, indem der Rotor bei einer Eigenfrequenz betrieben wird und die dadurch hervorgerufene Auslenkung des Rotors ermittelt wird. Mit Hilfe der Auslenkung des Rotors wird dann die Flüssigkeitsdämpfung ermittelt.

In einer dritten Variante wird die Flüssigkeitsdämpfung ermittelt, indem die Drehzahl des Rotors mit verschiedenen Frequenzen geändert wird und die bei der jeweiligen Drehzahländerungsfrequenz auftretende Änderung der axialen Lagerkräfte bzw. der axialen Auslenkung ermittelt wird. Aus der

Änderung der axialen Lagerkräfte bzw. der axialen Auslenkung des Rotors bei verschiedenen Drehzahländerungsfrequenzen wird dann die Flüssigkeitsdämpfung ermittelt.

Schliesslich wird in einer vierten Variante die Flüssigkeitsdämpfung ermittelt, indem die Verschiebung des Stabilitätsrands des Regelkreises für die axiale magnetische Lagerung des Rotors ermittelt wird. Aus dieser Verschiebung des Stabilitätsrands des Regelkreises für die axiale magnetische Lagerung wird dann die Flüssigkeitsdämpfung ermittelt.

Ist die Viskosität einmal bestimmt, so kann, wie bereits weiter oben erwähnt, bei denjenigen Verfahrensvarianten, bei denen der Durchfluss aus dem zuvor bestimmten Druck bestimmt wird (über die "Drosselkurve") bzw. bei denen der Durchfluss aus dem Drehmoment und der Drehzahl bestimmt wird (über die Leistungs-Fluss-Kennlinien), die ermittelte Viskosität berücksichtigt werden, wodurch die Genauigkeit der Werte für den Durchfluss bzw. den Druck noch weiter erhöht wird.

Im folgenden wird die Erfindung anhand der Zeichnung erläutert. Dabei zeigen in schematischer Darstellung:

Fig. 1    eine Darstellung des Zusammenhangs zwischen dem Druck und der axialen Auslenkung des Pumpenrotors,

Fig. 2    eine Dastellung des Zusammenhangs zwischen dem Druck und dem Durchfluss in einer Pumpe ("Drosselkurve")

Fig. 3    ein Ausführungsbeispiels einer axial passiven Lagerung eines Pumpenrotors mit Positionssensoren zur Bestimmung der Position des Rotors,

Fig. 4    einen Schnitt entlang der Linie IV-IV in Fig. 3,

Fig. 5    eine Darstellung analog Fig. 4, jedoch mit axial ausgelenktem Rotor,

Fig. 6    ein Beispiel für den Verlauf des magnetischen Flusses durch Positionssensoren zur Bestimmung der Position des Rotors,

Fig. 7    den prinzipiellen Verlauf der Amplitude des magnetischen Flusses durch einen Positionssensor in Abhängigkeit von der axialen Auslenkung des Rotors,

Fig. 8    ein Ausführungsbeispiel für eine axial versetzte Anordnung der Positionssensoren,

Fig. 9    ein Beispiel für den Verlauf des magnetischen Flusses bei einer Anordnung mit axial versetzten Positionssensoren,

Fig. 10    der prinzipielle Verlauf des Antriebsmoments in Abhängigkeit vom Antriebsstrom bei verschiedenen axialen Auslenkungen des Rotors,

Fig.11    eine Darstellung des Zusammenhangs zwischen der Leistung und dem Durchfluss bei einer Pumpe ("Leistungs-Fluss-Kennlinien"),

Fig. 12    eine qualitative Darstellung der Amplitude der Auslenkung des Rotors bei sich ändernder Drehzahl bei zwei verschiedenen Viskositäten der zu fördernden Flüssigkeit

und

Fig. 13    eine Darstellung des Parametergebiets eines stabilen Regelkreises (PD-Regler) der axialen magnetischen Lagerung des Rotors.

Wie bereits einleitend erwähnt wirken aufgrund der unterschiedlichen Drücke auf der Einlasseite und der Auslasseite einer Pumpe (einer Zentrifugalpumpe, einer Diagonalpumpe oder einer Axialpumpe) - also zu beiden Seiten des Pumpenrotors - auf den Pumpenrotor Axialkräfte. Diese Axialkräfte sind abhängig von der Druckdifferenz zwischen den beiden Seiten des Pumpenrotors. Wegen dieser Abhängigkeit der Axialkräfte von der Druckdifferenz ist es möglich, mit Hilfe der Axialkräfte die Druckdifferenz - den "Druck" in der Pumpe - zu bestimmen. Dies ist bei Pumpen mit einer magnetischen Lagerung des Pumpenrotors deshalb gut möglich, weil bei magnetischer Lagerung des Pumpenrotors die auf den Pumpenrotor wirkenden Axialkräfte sehr genau bestimmt werden können.

Für eine axial aktive magnetische Lagerung gilt:

$$F = k_i \bullet i$$

mit

k_i = Konstante
i = Lagerstrom,

während für eine axial passive magnetische Lagerung gilt:

$$F = k_z \bullet z$$

mit

k_z = Konstatne
z = Auslenkung des Rotors in z-Richtung.

Die nachfolgenden Betrachtungen erfolgen anhand von passiven axialen magnetischen Lagerungen des Rotors, gelten aber im übertragenen Sinne auch für aktive axiale magnetische Lagerungen.

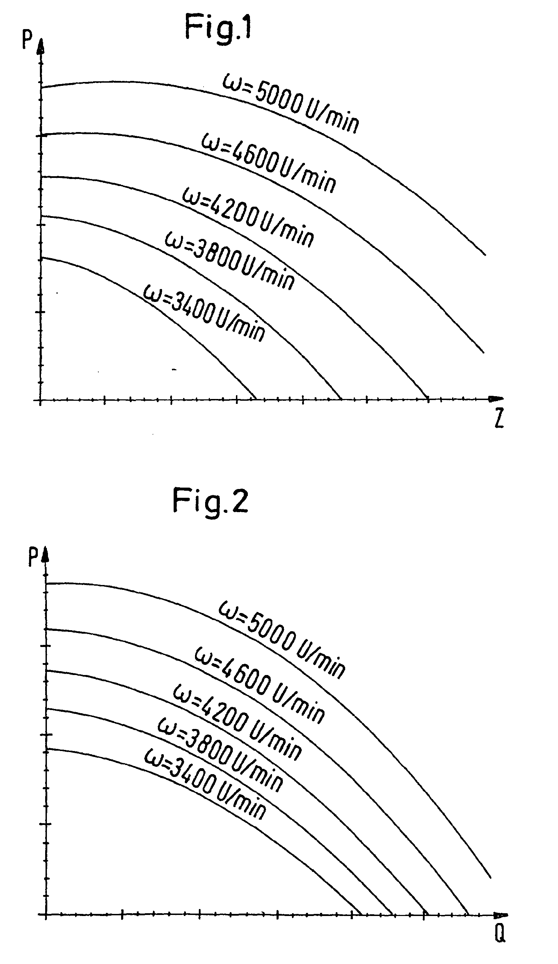

In Fig. 1 ist für eine passive axiale magnetische Lagerung des Rotors der Zusammenhang zwischen dem Druck p (Ordinate) und der Auslenkung z des Rotors in axialer Richtung (Abszisse) für verschiedene (Kreis)-Drehfrequenzen ω dargestellt. Im folgenden wird stets vereinfachend von der Drehzahl gesprochen, weil sich die Kreisdrehfrequenz aus der Drehzahl - multipliziert mit dem Faktor 2π - direkt ergibt. Die axiale Auslenkung z des Rotors ist der auf den Rotor wirkenden Axialkraft näherungsweise direkt proportional und somit ein Mass für die auf den Rotor wirkende Axialkraft.

Diese Abhängigkeit des Drucks p von der axialen Auslenkung z des Rotors bei verschiedenen Drehzahlen kann nun in Form einer elektronischen Nachschlagetabelle ("look-up-table") im Speicher eines Rechnners oder Mikroprozessors abgespeichert sein. Diese Nachschlagetabelle muss für den jeweiligen Pumpentyp nur einmal aufgenommen werden, sofern die Herstellung der einzelnen Pumpenteile innerhalb vorgegebener Toleranzen bleibt. Sie kann allerdings für jede einzelne Pumpe separat aufgenommen werden, was die Genauigkeit verbessert, aber auch den Aufwand erhöht. Um dabei die Datenmenge zu reduzieren, können einige Stützstellen aufgenommen werden. Wenn dann im Betrieb Auslenkungen auftreten, die zwischen zwei Stützstellen liegen, kommen übliche Interpolationsverfahren zum Einsatz.

Alternativ zu der Nachschlagetabelle kann die Abhängigkeit des Drucks p von der axialen Auslenkung z des Rotors aber auch durch ein Polynom beschrieben werden. Die einzelnen Koeffizienten dieses Polynoms werden entsprechend aufgenommen, und im Betrieb wird mit Hilfe der Kreisdrehfrequenz ω und der axialen Auslenkung z der Druck p errechnet. Ein solches Polynom kann beispielsweise die Form

$$p = k_1\,\omega\ +\ k_2\,z\ +\ k_3\,\omega\,z\ +\ k_4\,\omega^2\ +\ k_5\,z^2\ +\ k_6\,\omega\,z^2\ +\ k_7\,\omega^2\,z\ +\ k_8\,\omega^2\,z^2$$

aufweisen,
mit

     $k_i$ = Koeffizienten
     $\omega$ = Kreisdrehfrequenz
     $z$ = axiale Auslenkung des Rotors.

Ein mit Hilfe dieses Polynoms achter Ordnung bestimmter Druck p stellt eine sehr gute Approximation für den tatsächlichen Druck dar, die Genauigkeit kann natürlich durch Erhöhung der Ordnung des Polynoms weiter verbessert werden, allerdings erhöht sich dadurch natürlich auch der Rechenaufwand.

Ist auf eine dieser Weisen (mittels Nachschlagetabelle oder mittels polynomialer Approximation) der Druck p in der Pumpe bestimmt worden, so kann anschliessend mit Hilfe der Druck-Durchfluss-Kennlinie ("Drosselkurve") der Pumpe dann der Durchfluss Q durch die Pumpe bestimmt werden. Eine solche Drosselkurve ist in Fig. 2 dargestellt, man kann ihr den Zusammenhang zwischen Druck p in der Pumpe (Ordinate) und Durchfluss Q (Abszisse) bei verschiedenen Drehzahlen $\omega$ (bzw. Kreisdrehfrquenzen) entnehmen.

Die Drosselkurve kann entweder wieder in Form einer Nachschlagetabelle - wie oben beschrieben - abgespeichert sein, oder der Durchfluss kann bei bekanntem Druck durch ein Polynom errechnet werden (wie oben beschrieben). Der Vorteil dieses Verfahrens besteht natürlich einerseits darin, dass Druck p und Durchfluss Q sensorfrei bestimmt werden können. Der Hauptvorteil besteht aber darin, dass man das Antriebsdrehmoment des Pumpenrotors (bzw. den Motorstrom, der diesem Antriebsdrehmoment proportional ist) nicht kennen muss. Dies ist insofern ein grosser Vorteil, als sehr viele Antriebsmotoren für Pumpen entweder mit einer festen Drehspannung versorgt werden oder - bei Umrichterbetrieb - spannungsgesteuert betrieben werden, sodass der Motorstrom gar nicht gemessen wird und somit dem Antriebsregler als Messgrösse nicht bekannt ist.

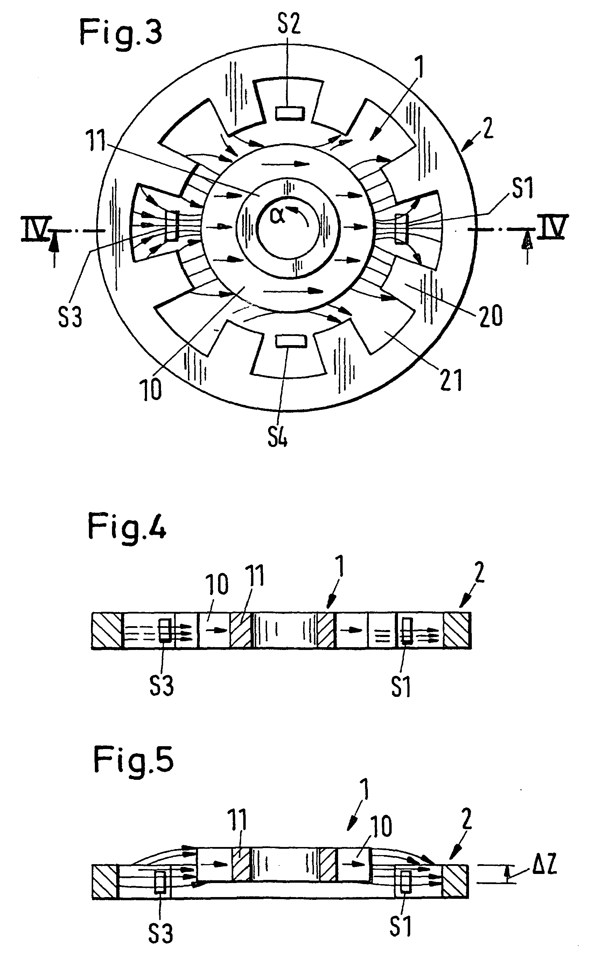

Nachfolgend wird eine weitere Ausführungsvariante beschrieben. Hierzu ist in Fig. 3 ein Ausführungsbeispiel einer axial passiven Lagerung eines Pumpenrotors am Beispiel eines lagerlosen Motors dargestellt, Fig. 4 zeigt einen Schnitt entlang der Linie IV-IV durch das Ausführungsbeispiel gemäss Fig. 3. Auch der Feldverlauf ist in diesen beiden Figuren qualitativ angedeutet. Man erkennt einen Pumpenrotor 1, der einen ringförmigen Magneten 10 sowie einen Eisenrückschluss 11 umfasst. Der Rotor 1 ist von einem Stator 2 umgeben, der hier mehrere Zähne 20 mit dazwischenliegenden Nuten 21 aufweist. Die Pfeile im ringförmigen Magneten 10 geben die Magnetisierungsrichtung an, die übrigen Pfeile stehen qualitativ für den Verlauf des Magnetfeldes bei dieser Rotorstellung.

Bei diesem Ausführungsbeispiel sind vier Positionssensoren S1,S2,S3,S4 jeweils in Nuten 21 zwischen Zähnen 20 angeordnet. Durch die Zähne 20 fliesst ein magnetischer Steuerfluss, der von Wicklungen (nicht dargestellt) herrührt, die um die Zähne 20 herum angeordnet sind, und der zur Steuerung der Rotorposition dient. Mit Hilfe der Sensoren S1,S2,S3,S4 lässt sich die Richtung und Stärke des Magnetfelds im Luftspalt und somit der Drehwinkel des Rotors 1 ermitteln. Die Sensoren sind typischerweise als Hall-Sensoren ausgebildet.

Bei einer Ausführungsvariante der Erfindung wird nun das Signal der Sensoren S1,S2,S3,S4 genutzt, um die axiale Auslenkung des Rotors zu bestimmen. Mit der axialen Auslenkung des Rotors ändert sich nämlich die Amplitude des mit den Sensoren gemessenen magnetischen Flusses. Die Anordnung der Sensoren S1,S2,S3,S4 in Nuten 21 ist dabei insofern von Vorteil, als der zuvor erwähnte Steuerfluss zur Steuerung der Rotorposition nicht von den Sensoren mitgemessen wird.

Betrachtet man Fig. 5, in welcher der Pumpenrotor 1 um eine Strecke Az aus seiner Ruheposition ausgelenkt ist, so ist unmittelbar ersichtlich, dass bei gleicher Winkellage des Rotors 1 der vom Rotor 1 herrührende magnetische Fluss, der die Sensoren - hier die Sensoren S1 und S3 durchdringt, geringer ist als wenn der Rotor sich in seiner Ruheposition befindet. Das Sensorsignal ist daher schwächer.

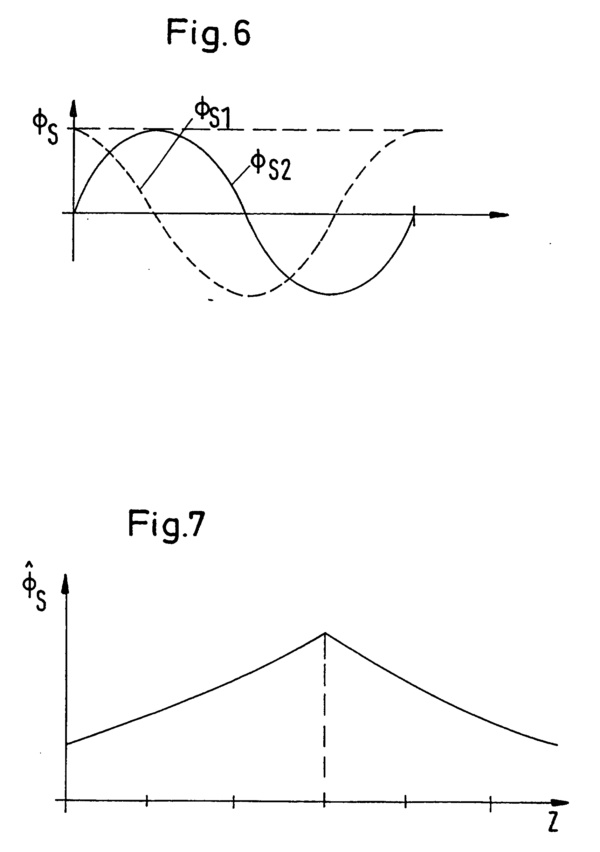

Prinzipiell lässt sich bereits mittels zwei Sensoren, die nicht um ein Vielfaches von 180° versetzt sind, beispielsweise mittels der Sensoren S1 und S2, sowohl die Winkellage als auch die axiale Position bzw. Auslenkung des Rotors 1 bestimmen. Ein Beispiel für den magnetischen Fluss $\phi_S$ durch die Sensoren S1,S2 (die Signale der Sensoren S3,S4 ergeben sich aus der Multiplikation der Signale der Sensoren S1,S2 mit dem Faktor -1) ist in Fig. 6 dargestellt.

Hierbei gelten folgende Zusammenhänge für den Winkel $\alpha$ und die Auslenkung z:

$$\alpha\ =\ \arctan\left(\phi_{S1}/\phi_{S2}\right)$$

und

$$z = f[(\phi_{S1}^2 + \phi_{S2}^2)^{1/2}],$$

wobei $\phi_{S1}$ und $\phi_{S2}$ den magnetischen Fluss durch die Sensoren S1 und S2 repräsentieren.

Falls vier Sensoren verwendet werden, wie im gezeigten Ausführungsbeispiel die Sensoren S1,S2,S3,S4, so lassen sich auf diese Weise die thermische Drift wie auch der Einfluss der Radialen Rotorposition (durch Differenzbildung) eliminieren. Für den Winkel $\alpha$ und die axiale Auslenkung z des Rotors gilt dann entsprechend:

$$\alpha = \arctan[(\phi_{S2} - \phi_{S4})/(\phi_{S1} - \phi_{S3})]$$

und

$$z = f[((\phi_{S1} - \phi_{S3})^2 + (\phi_{S2} - \phi_{S4})^2)^{1/2}]$$

In Fig. 7 ist nun ein prinzipieller Verlauf der Amplitude $\phi_S$ des magnetischen Flusses $\phi_S$ durch einen Sensor dargestellt. Man erkennt aus dieser Darstellung, dass bei der Anordnung der Sensoren, wie sie in Fig. 4 bzw. in Fig. 5 gezeigt ist, dass die Funktion

$$z = f(\phi_S)$$

nicht umkehrbar eindeutig (eineindeutig) ist. Der magnetische Fluss ist maximal, wenn sich der Rotor 1 in seiner Ruheposition befindet (keine axiale Auslenkung) und wird sowohl für eine in axialer Richtung positive als auch für eine in axialer Richtung negative Auslenkung kleiner.

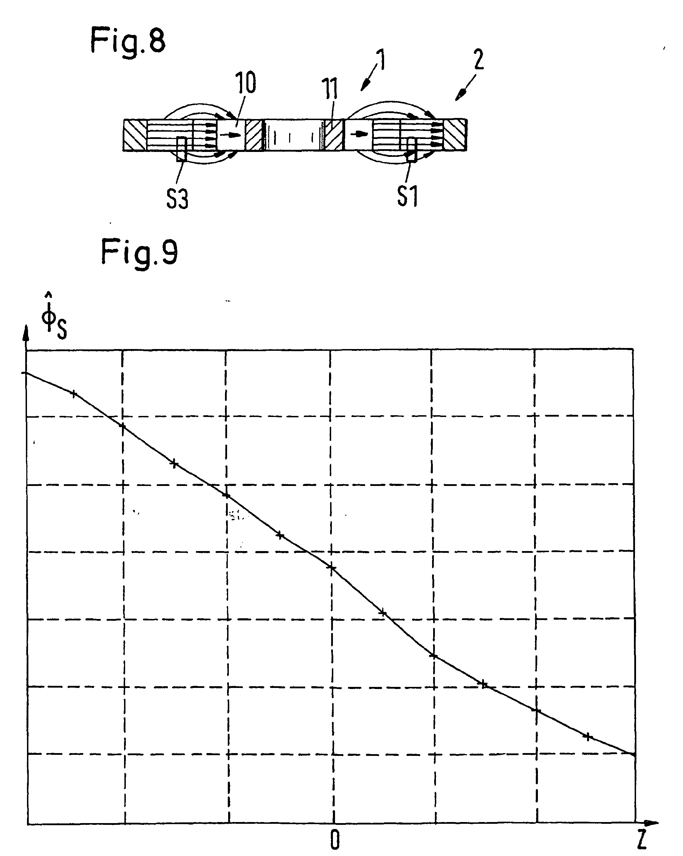

Um diesen Nachteil zu beseitigen kann man nun, wie in Fig. 8 anhand eines Ausführungsbeispiels gezeigt, die Sensoren - hier sind nur die Sensoren S1 und S3 zu erkennen, in axialer Richtung gegenüber der Ruheposition des Rotors 1 versetzt anordnen, in Fig. 8 nach unten.

Fig. 9 zeigt den Verlauf des magnetischen Flusses durch die Sensoren bei einer solchen Anordnung mit axial versetzt angeordneten Sensoren. Man erkennt erstens, dass die funktionelle Abhängigkeit des magnetischen Flusses durch die Sensoren nun umkehrbar eindeutig (eineindeutig) ist, und zweitens, dass der Zusammenhang bereits einigermassen linear ist, was aber nicht Voraussetzung ist. Das Sensorsignal wird maximal für eine negative Auslenkung und minimal für eine positive Auslenkung. Die Bestimmung der axialen Rotorposition ist nun eindeutig aus den Sensorsignalen möglich. Der Verlauf der Abhängigkeit des magnetischen Flusses von der axialen Auslenkung des Rotors 1, insbesondere die leichte Nichtlinearität des Verlaufs, lässt sich mittels einer Nachschlagetabelle ("look-up-table") mit Hilfe eines Mikroprozessors leicht linearisieren.

Bei einer axialen Auslenkung wird jedoch auch der für die Drehmomentbildung wirksame Motorfluss $\phi_M$ kleiner und somit ändert sich bei einem gegebenen Antriebsstrom $I_A$ das Antriebsmoment M in Abhängigkeit von der axialen Auslenkung z des Rotors 1.

Es gilt:

$$M \sim \phi_M \cdot I_A$$

wobei gilt

$$\phi_M \;=\; f(z),$$

sodass folgt

$$M \;=\; f(z, I_A)$$

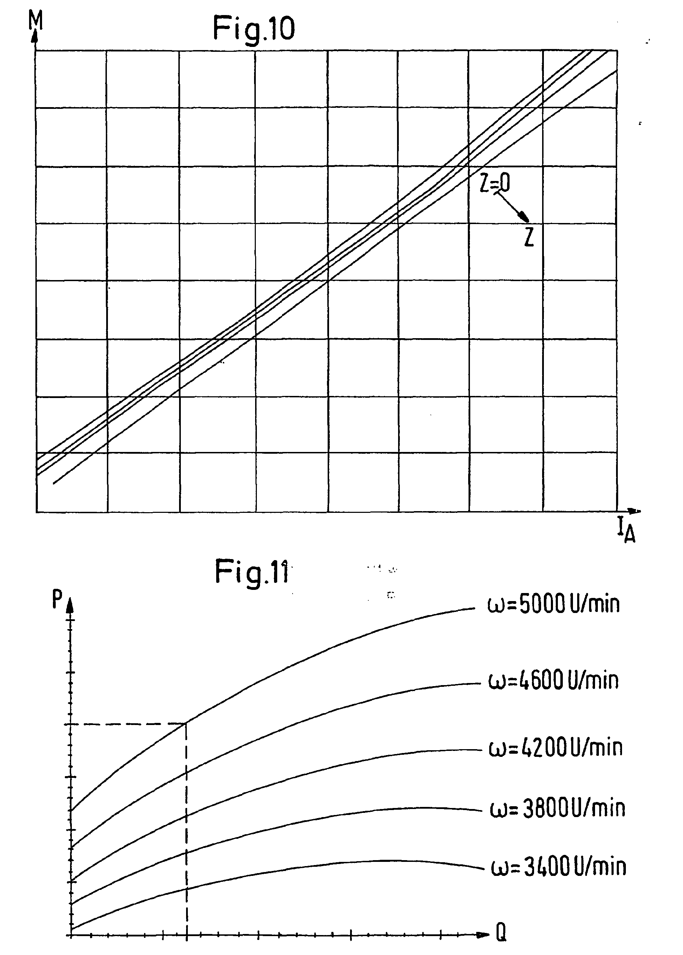

In Fig. 10 ist qualitativ ein solcher Verlauf des Antriebsmoments M in Abhängigkeit des Antriebsstroms $I_A$ gezeigt, wobei die einzelnen Geraden verschiedenen Auslenkungen des Rotors aus der Ruheposition entsprechen. Die oberste Gerade entspricht dem Zusammenhang zwischen Antriebsstrom $I_A$ und Antriebsmoment M in der Ruheposition des Rotors, die unterste Gerade dem Zusammenhang zwischen Antriebsstrom $I_A$ und dem Antriebsmoment M bei maximaler Auslenkung des Rotors. Man erkennt, dass der Zusammenhang zwischen dem Antriebsmoment M und dem Antriebsstrom $I_A$ auch bei einer axialen Auslenkung des Rotors aus der Ruheposition in hohem Masse linear bleibt.

Die Abhängigkeit des Motorflusses $\phi_M$ bzw. des Antriebsmoment M von der axialen Auslenkung z des Rotors kann nun wieder in einer Nachschlagetabelle ("look-up-table") abgespeichert sein. Auf diese Weise lässt sich der Einfluss der axialen Lage des Rotors bei der Bestimmung des tatsächlich wirksamen Antriebsmoments eliminieren und man erhält ein von der axialen Auslenkung des Rotors unabhängiges tatsächlich wirksames Antriebsmoment M.

Für die Leistung einer Pumpe gilt:

$$P = M \bullet \omega$$

mit

 M = Antriebsmoment

 $\omega$ = Kreisdrehfrequenz

Ist nun, wie oben beschrieben, das tatsächliche (also das von der axialen Position des Rotors unabhängige) Antriebsmoment M bekannt, so kann mit Hilfe der Leistungs-Fluss-Kennlinien der Durchfluss Q durch die Pumpe ermittelt werden.

Eine solche Leistungs-Fluss-Kurvenschar ist von ihrem prinzipiellen Verlauf für verschiedene (Kreis-)Drehzahlen $\omega$ in Fig. 11 dargestellt. Man erkennt auch hier, dass der Zusammenhang zwischen dem Durchfluss Q und der Leistung P nicht-linear ist. Ferner erkennt man aus dieser Darstellung auch, dass der lineare Zusammenhang zwischen Durchfluss Q und Antriebsmoment M in der eingangs bei der Diskussion des Stands der Technik genannten US-A-4,781,525 allenfalls in einem begrenzten Bereich Gültigkeit besitzt. Allgemein ist der Zusammenhang zwischen Durchfluss Q und Leistung P und damit auch der Zusammenhang zwischen Durchfluss Q und Antriebsmoment M nicht-linear.

Die Leistungs-Fluss-Kennlinien aus Fig. 11 für die Pumpe können wieder in Form einer Nachschlagetabelle ("look-up-table") gespeichert sein, wobei für einen Pumpentyp diese Kennlinien nur einmal aufgenommen werden müssen, sofern die Pumpenteile bei der Herstellung innerhalb vorgegebener Toleranzen bleiben. Alternativ kann auch hier eine polynomiale Approximation erfolgen, wie bereits weiter oben beschrieben.

Hat man über die Leistungs-Fluss-Kennlinien den Durchfluss Q durch die Pumpe bestimmt, so kann anschliessend über die Druck-Durchfluss-Kennlinie ("Drosselkurve") auch der Druck p in der Pumpe bestimmt werden. Der auf diese Weise berechnete Durchfluss Q durch die Pumpe und auch der auf diese Weise ermittelte Druck p sind nun aber stark von der Viskosität $\eta$ der zu fördernden Flüssigkeit abhängig:

$$Q_{real} \;=\; f_1\,(Q_{berechnet}\,,\,\eta)$$

$$p_{real} \;=\; f_2\,(p_{berechnet}\,,\,\eta)$$

Andererseits ist der aus der axialen Lagerkraft bestimmte Druck p in der Pumpe (siehe Variante, die anhand von Fig. 1 und Fig. 2 beschrieben ist) im wesentlichen unabhängig von der Viskosität η der Flüssigkeit.

Bestimmt man also zunächst über die Axialkraft (also bei aktiven axialen Lagerungen des Rotors über den Lagerstrom i, bei passiven axialen Lagerungen des Rotors über die axiale Auslenkung z) den Druck $p_{real}$ und anschliessend aus $p_{real}$, anschliessend mit Hilfe des Antriebsmoments M und der Drehzahl ω über die Leistung P der Pumpe und daraus mit Hilfe der Leistungs-Fluss-Kennlinien den Durchfluss Q und aus diesem dann dem Druck $P_{berechnet}$, so kann schliesslich aus den Werten für $P_{real}$ und $P_{berechriet}$ die Viskosität η ermittelt werden. Kennt man die Viskosität η der Flüssigkeit, so kann anschliessend die Bestimmung von Druck p und Durchfluss Q mit Hilfe der Leistungs-Fluss-Kennlinien, die stark von der Viskosität abhängig sind, exakter ermittelt werden.

Es soll hier angemerkt werden, dass die Bestimmung von Durchfluss Q und Druck p mittels des Leistungs-Fluss-Kennlinien ohne Berücksichtigung der Viskosität, also dann, wenn man bei einer Flüssigkeit eines bestimmten Typs von einer für diesen Flüssigkeitstyp üblichen Viskosität ausgeht, ebenfalls gute Ergebnisse liefert. Ist jedoch diese Genauigkeit nicht ausreichend oder wünscht man z.B. bei Blutpumpen eine sehr hohe Genauigkeit bei der Bestimmung von Druck p und Durchfluss Q, so kann zuvor eine Bestimmung der Viskosität η erfolgen.

Bei dem oben beschriebenen Verfahren zur Bestimmung der Viskosität aus , $p_{berechnet}$ und $P_{real}$ liegen die dort erwähnten Funktionen $f_1$ und $f_2$ sehr pumpenspezifisch sind und normalerweise nur in Form von gemessenen Kennlininen vorliegen, nicht hingegen als mathematische Funktionen vorliegen. Die Kennlinien werden dann zur mathematischen Behandlung vorzugsweise mit Hilfe von Poylnomen angenähert.

Physikalisch betrachtet ist die Viskosität η ein Mass für die (geschwindigkeits-proportionale) Flüssigkeitsreibung. Folglich hat sie die Form einer Dämpfungskonstante und es gilt:

$$F_R \sim \eta \bullet v$$

mit

$F_R$ = Reibungskraft
v = Geschwindigkeit
η = Viskosität.

Andererseits kann jedes Magnetlager durch seine Steifigkeit $S_{ML}$ und durch seine Dämpfung $D_{ML}$ charakterisiert werden. Befindet sich nun der magnetgelagerte Rotor in einer Flüssigkeit, so wird im Vergleich zum Betrieb in Luft vor allem die Dämpfung vergrössert. Es gilt:

$$D_{ges} = D_{ML} + D_{FL}$$

Durch dirkete Ermittlung der Flüssigkeitdämpfung $D_{FL}$ oder durch Ermittlung der Gesamtdämpfung $D_{ges}$ des Systems lässt sich dann ein Mass für die Viskosität finden, da sich die Gesamtdämpfung ja additiv aus der Dämpfung des Magnetlagers und der Flüssigkeitsdämpfung zusammensetzt. Im folgenden werden verschiedene Variante beschrieben, wie sich die Systemdämpfung $D_{ges}$ oder direkt die Flüssigkeitdämpfung $D_{FL}$ in einer Pumpe mit magnetgelagertem Rotor bestimmen lassen.

Bei einer ersten Variante wird die Flüssigkeitsdämpfung mit Hilfe der Verschiebung der Eigenfrequenzen des Rotors ermittelt. Eigenschwingungen des Körpers können unterteilt werden in sogenannte Starrkörperschwingungen, d.h. der Körper behält seine äussere Form bei, und in Biegeschwingungen. Die Eigenfrequenzen der Starrkörperschwingungen werden vorgegeben durch die Masse und die Geometrie des Rotors und durch die Magnetlagerparameter Steifigkeit $S_{ML}$ und $D_{ML}$. Bei den Biegeschwingungen kommt noch die Materialdämpfung des Rotors hinzu, da sich der Rotor dabei ja verformt.

Die Eigenfrequenzen lassen sich nun auf verschiedene Arten bestimmen:

a) Durch vorzugsweise in radialer Richtung gerichtete breitbandige Anregung des Rotors (z.B. mit Hilfe eines Rechtecksignals oder eines Chirpsignals) mit Hilfe des Magnetlagers und durch spektrale Analyse der Signale der (radialen) Positionssignale.

b) Durch (axiale) Anregung des Rotors über ein aktives Magnetlager mit einem gleitenden Sinus oder durch sinusförmige Prüfsignale mit diskreten frequenzmässigen Stufen und Aufzeichnung der Rotorauslenkung (Frequenzgangmessung)

c) Durch Anregung der Eigenfrequenzen mittels drehzahlsynchroner Störkräfte (wie z.B. Unwucht, hydraulische Unwucht, Schaufelkräfte), wobei die Eigenfrequenzen durchfahren werden und die radiale Auslenkung des Rotors (Orbit) analysiert wird.

Die Eigenfrequenzen des Rotors in Luft sind entweder bekannt oder werden ermittelt. Hat man dann die Eigenfrequenzen des Rotors in der Flüssigkeit auf eine der beschriebenen Arten ermittelt, so kann der Zusammenhang zwischen der Verschiebung der Eigenfrequenzen und die daraus resultierende Viskosität $\eta$ in z.B. in einer Nachschlagetabelle ("look-up-table") abgespeichert sein.

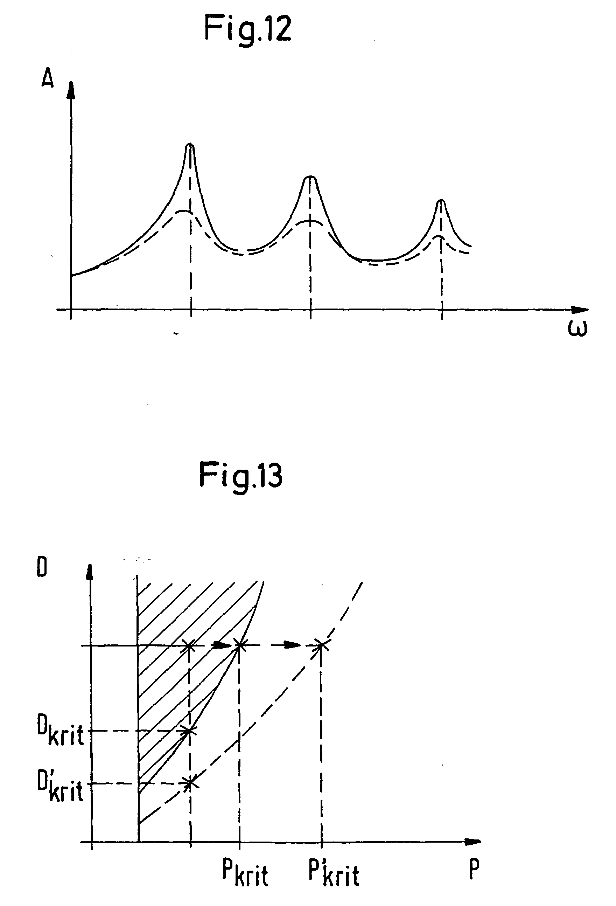

Bei einer zweiten Variante wird der Rotor versuchsweise mit einer Drehzahl betrieben, die einer Eigenfrequenz des Rotors entspricht. Um eine Eigenfrequenz des Rotors zu finden, wird am besten die Drehzahl oder die Magnetlagersteifigkeit $S_{ML}$ variiert. Die Amplitude A der Auslenkung des Rotors (Grösse des Orbits) ist dann ein Mass für die Systemdämpfung $D_{ges}$, aus der dann die Flüssigkeitsdämpfung $D_{FL}$ und aus dieser die Viskosität $\eta$ ermittelt werden kann (siehe z.B. Fig.12, die gestrichelte Linie repräsentiert die Flüssigkeit mit der höheren Viskosität $\eta$). Die Amplitude der Auslenkung und die jeweilige daraus folgende Viskosität $\eta$ können dann in einer Nachschlagetabelle ("look-up-table") abgespeichert sein, sodass aus der jeweilige Amplitude der Auslenkung dann die Viskosität $\eta$ bestimmt werden kann.

Bei einer dritten Variante wird die Drehzahl um einen vorgegebenen Betrag variiert, beispielsweise um einen Betrag von $\pm 10\%$. Mit einer Änderung der Drehzahl ändert sich auch die Axiallagerkraft, bei einem passiven Magnetlager ändert sich die axiale Auslenkung des Rotors. Bei einer niedrigen Drehzahländerungsfrequenz ist die axiale Auslenkung weniger abhängig von der Viskosität, bei einer hohen Drehzahländerungsfrequenz ist diese Abhängigkeit stärker. Aus der Messung bei zwei oder mehreren Drehzahländerungsfrequenzen lässt sich dann die Flüssigkeitsdämpfung $D_{FL}$ und aus dieser die Viskosität $\eta$ ermitteln.

Dieser Zusammenhang zwischen der Änderung der Axiallagerkraft bei zwei oder mehreren Drehzahländerungsfrequenzen bzw. zwischen der axialen Auslenkung des Rotors bei zwei oder mehreren Drehzahländerungsfrequenzen und die daraus resultierende Viskosität $\eta$ kann dann beispielsweise in einer Nachschlagetabelle ("look-up-table") abgespeichert sein.

Bei einer vierten Variante wird die Verschiebung des Stabilitätsrandes des geschlossenen Regelkreises für die axiale magnetische Lagerung des Rotors ermittelt. Das Parametergebiet, in welchem ein stabiler Betrieb des Regelkreises möglich ist, wird im wesentlichen durch dessen Verstärkung (den P-Anteil) und durch dessen Dämpfung (D-Anteil) bestimmt. In Fig. 13 ist ein solches Parametergebiet, in welchem ein stabiler Betrieb des Regelkreises möglich ist, für einen einfachen PD-Regler beispielhaft dargestellt. Befindet sich der Rotor in Luft, so ist ein stabiler Betrieb des Regelkreises innerhalb des schraffierten Parametergebiets möglich.

Durch eine Vergrösserung der Verstärkung des Regelkreises (Vergrösserung des P-Anteils) auf die kritische Verstärkung $P_{krit}$ hin oder durch eine Verkleinerung der Dämpfung auf die kritische Dämpfung $D_{krit}$ hin lässt sich ein grenzstabiles Verhalten erreichen, d.h. der Rotor beginnt bezüglich derjenigen Reglerachse (es gibt fünf solcher Achsen: die zwei Achsen horizontaler Verschiebung des Rotors in der radialen Lagerebene, die zwei Kippachsen und die Achse der Verschiebung des Rotors senkrecht zur Lagerebene), in welcher die Parameterverschiebung erfolgt, Dauerschwingungen auszuführen. Durch eine kurzzeitige Erhöhung der Verstärkung des Regelkreises (P-Anteil) bzw. durch eine kurzzeitige Verringerung der Dämpfung (D-Anteil) kann der Stabilitätsrand gefunden werden, indem beobachtet wird, wann die Dauerschwingungen des Rotors bezüglich der entsprechenden Reglerachse einsetzen.

Befindet sich nun der Rotor nicht in Luft, sondern in einer Flüssigkeit, so verschiebt sich die kritische Dämpfung $D_{knt}$ des Regelkreises selbst in Richtung einer geringeren kritischen Dämpfung $D'_{krit}$, weil ja durch die Flüssigkeit eine zusätzliche Dämpfung bewirkt wird. Die kritische Verstärkung $P_{krit}$ des Regelkreises selbst hingegen verschiebt sich in Richtung einer höheren kritischen Verstärkung $P'_{krit}$, weil ein Teil der Verstärkung von der zusätzlichen Dämpfung der Flüssigkeit "aufgefressen" wird. Die Verschiebung des Stabilitätsrandes des Regelkreises für die axiale magnetische Lagerung des Rotors ist daher ein Mass für die Flüssigkeitsdämpfung $D_{FL}$, aus der dann die Viskosität $\eta$ der Flüssigkeit bestimmt werden kann.

Ist auf eine der beschriebenen Arten die Viskosität $\eta$ der Flüssigkeit bestimmt worden, so kann - wie bereits weiter oben beschrieben - mit Hilfe der so bestimmten Viskosität $\eta$ die Bestimmung des Durchflusses Q aus den Leistungs-Fluss-Kennlinien (siehe Fig. 11) und die anschliessende Bestimmung des Drucks p aus den Druck-Durchfluss-Kennlinien ("Drosselkurven") bzw. die Bestimmung des Durchflusses Q aus den Druck-Durchfluss-Kennlinien ("Drosselkurven") bei zuvor mit Hilfe der Axialkräfte bestimmtem Druck p noch exakter erfolgen, weil die jeweilige Viskosität $\eta$ der Flüssigkeit berücksichtigt werden kann.

**Patentansprüche**

1. Verfahren zur Bestimmung des Drucks (p) und/oder des Durchflusses (Q) einer Flüssigkeit in bzw. durch eine Zentrifugalpumpe oder Diagonalpumpe oder Axialpumpe, welche mindestens ein Magnetlager zur axialen Lagerung des Pumpenrotors (1) aufweist, wobei die axiale magnetische Lagerung des Pumpenrotors als aktive oder passive Lagerung ausgebildet ist, und wobei beim Betrieb auf Grund des Drucks in der Pumpe auf den Rotor (1) Axialkräfte wirken, **dadurch gekennzeichnet, dass** zur Bestimmung des Drucks (p) und/oder des Durchflusses (Q) die auf den Rotor wirkenden Axialkräfte ermittelt werden, und dass zur Ermittlung der auf den Rotor (1) wirkenden Axialkräfte bei aktiver Ausbildung der axialen magnetischen Lagerung der zur axialen Lagerung des Rotors (1) erforderliche Lagerstrom und bei passiver Ausbildung der axialen magnetischen Lagerung die axiale Auslenkung (z,$\Delta$z) des Rotors (1) herangezogen wird.

2. Verfahren nach Anspruch 1, **dadurch gekennzeichnet, dass** verschiedene zur axialen Lagerung des Rotors (1) erforderliche Lagerströme bzw. verschiedene axiale Auslenkungen (z,$\Delta$z) des Rotors (1) jeweils bei verschiedenen Drehzahlen ($\omega$) sowie die jeweiligen daraus resultierenden Drücke in einer elektronischen Nachschlagetabelle abgespeichert sind, sodass beim Betrieb der Pumpe der jeweilige Lagerstrom bzw. die jeweilige axiale Auslenkung (z,$\Delta$z) des Rotors (1) sowie seine Drehzahl ($\omega$) ermittelt werden, und dann mit Hilfe dieser Nachschlagetabelle der daraus resultierende Druck (p) bestimmt wird.

3. Verfahren nach Anspruch 1, **dadurch gekennzeichnet, dass** der Druck (p) in der Pumpe aus dem jeweiligen zur axialen Lagerung des Rotors erforderlichen Lagerstrom bzw. aus der jeweiligen axialen Auslenkung (z,$\Delta$z) des Rotors (1) einerseits sowie aus der jeweiligen Drehzahl ($\omega$) andererseits mit Hilfe eines Polynoms bestimmt wird.

4. Verfahren nach einem der Ansprüche 2 oder 3, **dadurch gekennzeichnet, dass** aus dem bereits bestimmten Druck (p) und der zugehörigen Drehzahl ($\omega$) dann der Durchfluss (Q) durch die Pumpe über die Druck-Durchfluss-Kennlinien der Pumpe bestimmt wird.

5. Verfahren nach Anspruch 4, **dadurch gekennzeichnet, dass** die Druck-Durchfluss-Kennlinien der Pumpe in einer elektronischen Nachschlagetabelle abgespeichert sind, sodass beim Betrieb der Pumpe aus dem bereits bestimmten Druck (p) dann mit Hilfe dieser Nachschlagetabelle der Durchfluss (Q) durch die Pumpe bestimmt wird.

6. Verfahren nach Anspruch 4, **dadurch gekennzeichnet, dass** die Druck-Durchfluss-Kennlinien durch ein Polynom approximiert werden, sodass beim Betrieb der Pumpe aus dem bereits bestimmten Druck (p) mit Hilfe dieses Polynoms dann der Durchfluss (Q) durch die Pumpe bestimmt wird.

7. Verfahren nach einem der Ansprüche 1 bis 6, **dadurch gekennzeichnet, dass** eine Pumpe mit Sensoren (S1,S2,S3,S4) zur Bestimmung der Richtung des Magnetfelds im Spalt zwischen dem Rotor (1) und dem Stator (2) verwendet wird und dass diese Sensoren (S1,S2,S3,S4) auch zur Bestimmung der axialen Auslenkung (z,$\Delta$z) des Rotors (1) verwendet werden.

8. Verfahren nach Anspruch 7, **dadurch gekennzeichnet, dass** der Durchfluss (Q) durch die Pumpe bestimmt wird, indem das den Rotor (1) antreibende Drehmoment (M) und die Drehzahl ($\omega$) des Rotors (1) ermittelt werden, wobei zur Ermittlung des Drehmoments (M) einerseits der magnetische Antriebsfluss ($\phi_M$) im Spalt zwischen Rotor (1) und Stator (2) sowie der Antriebsstrom ($I_A$) für den Rotor (1) ermittelt werden und wobei andererseits die axiale Auslenkung (z,$\Delta$z) des Rotors (1) ermittelt wird und aus dem Antriebsstrom ($I_A$) und der axialen Auslenkung (z,$\Delta$z) des Rotors (1) dann das antreibende Drehmoment (M) ermittelt wird, sodass schliesslich aus dem so ermittelten Drehmoment (M) und der ermittelten Drehzahl ($\omega$) der Durchfluss (Q) durch die Pumpe bestimmt wird.

9. Verfahren nach Anspruch 8, **dadurch gekennzeichnet, dass** verschiedene Antriebsströme ($I_A$) bei verschiedenen axialen Auslenkungen des Rotors sowie das jeweils daraus resultierende Drehmoment (M) in einer elektronischen Nachschlagetabelle abgespeichert sind, sodass beim Betrieb der Pumpe aus der jeweiligen axialen Auslenkung (z, $\Delta$z) des Rotors (1) und dem jeweiligen Antriebsstrom ($I_A$) zunächst mit Hilfe dieser Nachschlagetabelle das Drehmoment (M) ermittelt wird und anschliessend aus dem so ermittelten Drehmoment (M) und der ermittelten Drehzahl ($\omega$) dann der Durchfluss (Q) durch die Pumpe bestimmt wird.

10. Verfahren nach Anspruch 8 oder 9, **dadurch gekennzeichnet, dass** aus dem bereits bestimmten Durchfluss (Q) und der zugehörigen Drehzahl ($\omega$) über die Druck-Durchfluss-Kennlinien der Pumpe der Druck (p) in der Pumpe bestimmt wird.

**11.** Verfahren nach Anspruch 10, **dadurch gekennzeichnet, dass** die Druck-Durchfluss-Kennlinien der Pumpe in einer elektronischen Nachschlagetabelle abgespeichert sind, sodass beim Betrieb der Pumpe aus dem bereits bestimmten Durchfluss (Q) dann mit Hilfe dieser Nachschlagetabelle der Druck (p) in der Pumpe bestimmt wird.

**12.** Verfahren nach Anspruch 10, **dadurch gekennzeichnet, dass** die Druck-Durchfluss-Kennlinien durch ein Polynom approximiert werden, sodass beim Betrieb der Pumpe aus dem bereits bestimmten Durchfluss (Q) mit Hilfe dieses Polynoms dann der Druck (p) in der Pumpe bestimmt wird.

**13.** Verfahren nach einem der Ansprüche 4 bis 12, **dadurch gekennzeichnet, dass** zur Bestimmung des Durchflusses (Q) aus dem zuvor bestimmten Druck (p) bzw. zur Bestimmung des Durchflusses (Q) aus dem Drehmoment (M) und der Drehzahl ($\omega$) die Viskosität ($\eta$) der Flüssigkeit in der Pumpe ermittelt wird.

**14.** Verfahren nach Anspruch 13, **dadurch gekennzeichnet, dass** zur Ermittlung der Viskosität ($\eta$) der Flüssigkeit der mit Hilfe der auf den Rotor (1) wirkenden Axialkräfte bestimmte Druck (p) verglichen wird mit dem Druck (p), der sich aus der Bestimmung mit Hilfe des ermittelten Drehmoments (M) und der ermittelten Drehzahl ($\omega$) ergibt, und dass dann aus der Differenz dieser beiden Drücke und der zugehörigen Drehzahl die Viskosität ($\eta$) bestimmt wird.

**15.** Verfahren nach Anspruch 13, **dadurch gekennzeichnet, dass** zur Bestimmung der Viskosität ($\eta$) der Flüssigkeit die auf den Rotor (1) wirkende Flüssigkeitsdämpfung ($D_{FL}$) ermittelt wird und aus der ermittelten Flüssigkeitsdämpfung ($D_{FL}$) dann die Viskosität ($\eta$) bestimmt wird.

**16.** Verfahren nach Anspruch 15, **dadurch gekennzeichnet, dass** die Flüssigkeitsdämpfung ($D_{FL}$) mit Hilfe der Verschiebung der Eigenfrequenzen des Rotors (1) ermittelt wird.

**17.** Verfahren nach Anspruch 15 **dadurch gekennzeichnet, dass** die Flüssigkeitsdämpfung ($O_{FL}$) ermittelt wird, indem der Rotor (1) bei einer Eigenfrequenz betrieben wird und die **dadurch** hervorgerufene Auslenkung des Rotors ermittelt wird, mit deren Hilfe dann die Flüssigkeitsdämpfung ($D_{FL}$) ermittelt wird.

**18.** Verfahren nach Anspruch 15, **dadurch gekennzeichnet, dass** die Flüssigkeitsdämpfung ($D_{FL}$) ermittelt wird, indem die Drehzahl ($\omega$) des Rotors (1) mit verschiedenen Frequenzen geändert wird und die bei der jeweiligen Drehzahländerungsfrequenz auftretende Änderung der axialen Lagerkräfte bzw. der axialen Auslenkung ermittelt wird, und dass aus der Änderung der axialen Lagerkräfte bzw. der axialen Auslenkung des Rotors (1) bei verschiedenen Drehzahländerungsfrequenzen dann die Flüssigkeitsdämpfung ($D_{FL}$) ermittelt wird.

**19.** Verfahren nach Anspruch 15, **dadurch gekennzeichnet, dass** die Flüssigkeitsdämpfung ($D_{FL}$) ermittelt wird, indem die Verschiebung des Stabilitätsrands des Regelkreises für die axiale magnetische Lagerung des Rotors (1) ermittelt wird, und aus dieser Verschiebung dann die Flüssigkeitsdämpfung ($D_{FL}$) ermittelt wird.

**20.** Verfahren nach einem der Ansprüche 4 bis 12 und nach einem der Ansprüche 13 bis 19, **dadurch gekennzeichnet, dass** zur Bestimmung des Durchflusses (Q) aus dem zuvor bestimmten Druck (p) bzw. zur Bestimmung des Durchflusses (Q) aus dem Drehmoment (M) und der Drehzahl ($\omega$) die ermittelte Viskosität ($\eta$) berücksichtigt wird.


**Claims**

**1.** A method for the determination of the pressure (p) and/or the through-flow (Q) of a liquid in and/or through a centrifugal pump or a diagonal pump or an axial pump which has at least one magnetic bearing for the axial support of the pump rotor (1), wherein the axial magnetic support of the pump rotor is configured as an active or passive support and wherein, on operation, axial forces act on the rotor (1) due to the pressure in the pump, **characterized in that** for the determination of the pressure (p) and/or of the through-flow (Q) the axial forces acting on the rotor are determined and **in that** for the determination of the axial forces acting on the rotor with an active design of the axial magnetic bearing the support current required for the axial support of the rotor (1) is used and with a passive design of the axial magnetic bearing the axial deflection (z, $\Delta$z) of the rotor (1) is used.

**2.** A method in accordance with claim 1, **characterized in that** different support currents required for the axial support of the rotor (1) and/or different axial deflections (z, $\Delta$z) of the rotor (1) are stored in an electronic look-up table for respectively different rotational speed ($\omega$) as well as the pressure respectively resulting therefrom so that on operation of the pump the respective support current and/or the respective axial deflection (z, $\Delta$z) of the rotor (1) as well as

its rotational speed ($\omega$) are determined and then the thereby resulting pressure (p) is determined with the aid of this look-up table.

3. A method in accordance with claim 1, **characterized in that** the pressure (p) in the pump is determined from the respective support current required for the axial support of the rotor and/or from the respective axial deflection (z, $\Delta z$) of the rotor (1), on the one hand, and from the respective rotational speed ($\omega$), on the other hand, with the aid of a polynomial.

4. A method in accordance with one of the claims-2 or 3, **characterized in that** the through-flow (Q) through the pump is then determined via the pressure through-flow characteristic curve from the already determined pressure (p) and the associated rotational speed ($\omega$).

5. A method in accordance with claim 4, **characterized in that** the pressure through-flow characteristic curve of the pump is stored in an electronic look-up table so that on operation of the pump the through-flow (Q) through the pump is then determined from the already determined pressure (p) with the aid of this look-up table.

6. A method in accordance with claim 4, **characterized in that** the pressure through-flow characteristic curve is approximated by a polynomial so that on operation of the pump the through-flow (Q) through the pump is then determined from the already determined pressure (p) with the aid of this polynomial.

7. A method in accordance with any one of the claims 1 to 6, **characterized in that** a pump having sensors (S1, S2, S3, S4) is used for the determination of the direction of the magnetic field in the gap between the rotor (1) and the stator (2) and that these sensors (S1, S2, S3, S4) are also used for the determination of the axial deflection (z, $\Delta z$) of the rotor (1).

8. A method in accordance with claim 7, **characterized in that** the through-flow (Q) through the pump is determined **in that** the drive torque (M) driving the rotor (1) and the associated rotational speed ($\omega$) of the rotor (1) are determined, wherein for the determination of the drive torque (M) , on the one hand, the magnetic drive flux ($\phi_M$) is determined in the gap between the rotor (1) and stator (2) and also the drive current ($I_A$) for the rotor (1) is determined and wherein, on the other hand, the axial deflection (z, $\Delta z$) of the rotor (1) is determined and from the drive current ($I_A$) and the axial deflection (z, $\Delta z$) of the rotor (1) then the driving drive torque (M) is determined so that subsequently the through-flow (Q) through the pump is determined from the thus determined drive torque (M) and the determined associated rotational speed ($\omega$).

9. A method in accordance with claim 8, **characterized in that** different drive currents ($I_A$) for different axial deflections of the rotor as well as the thereby resulting respective drive moments (M) are stored in an electronic look-up table so that to begin with, on operation of the pump, the drive moment (M) is determined from the respective axial deflection (z, $\Delta z$) of the rotor (1) and the respective drive current ($I_A$) with the aid of this look-up table and subsequently the through-flow (Q) through the pump is then determined from the thus determined drive element (M) and the determined associated rotational speed ($\omega$).

10. A method in accordance with claim 8 or 9, **characterized in that** from the already determined through-flow (Q) and the associated rotational speed ($\omega$), the pressure (p) in the pump is determined via the pressure through-flow characteristic curve of the pump.

11. A method in accordance with claim 10, **characterized in that** the pressure through-flow characteristic curve of the pump is stored in an electronic look-up table so that on operation of the pump the pressure (p) in the pump is then determined from the already determined through-flow (Q) with the aid of this look-up table.

12. A method in accordance with claim 10, **characterized in that** the pressure through-flow characteristic curve is approximated by a polynomial so that on operation of the pump the pressure (p) in the pump is then determined from the already determined through-flow (Q) with the aid of this polynomial.

13. A method in accordance with any one of the claims 4 to 12, **characterized in that** for the determination of the through-flow (Q) from the previously determined pressure (p) and/or for the determination of the through-flow (Q) from the drive torque (M) and the associated rotational speed ($\omega$) the viscosity ($\eta$) of the liquid in the pump is determined.

**14.** A method in accordance with claim 13, **characterized in that** for the determination of the viscosity (η) of the liquid the pressure (p) determined with the aid of the axial forces acting on the rotor (1) is compared with the pressure (p) which results from the determination of the drive moment (M) and the determined associated rotational speed (ω) and **in that** the viscosity (η) is then determined from the difference of these two pressure and the associated rotational speed.

**15.** A method in accordance with claim 13, **characterized in that** for the determination of the viscosity (η) of the liquid the liquid damping ($D_{FL}$) acting on the rotor (1) is determined and from the determined liquid damping ($D_{FL}$) the viscosity (η) is then determined.

**16.** A method in accordance with claim 15, **characterized in that** the liquid damping ($D_{FL}$) is determined with the aid of the displacement of the eigen-frequencies of the rotor (1).

**17.** A method in accordance with claim 15, **characterized in that** the liquid damping ($D_{FL}$) is determined **in that** the rotor (1) is operated at an eigen-frequency and the thereby caused deflection of the rotor is determined with whose aid the liquid damping ($D_{FL}$) is then determined.

**18.** A method in accordance with claim 15, **characterized in that** the liquid damping ($D_{FL}$) is determined **in that** the associated rotational speed (ω) of the rotor (1) is changed with different frequencies and the change of the axial support forces and/or the axial deflection occurring on the respective rotational speed change frequencies are determined and **in that** the liquid damping ($D_{FL}$) is then determined from the change of the axial support forces and/or the axial deflection of the rotor (1) for different rotational speed change frequencies.

**19.** A method in accordance with claim 15, **characterized in that** the liquid damping ($D_{FL}$) is determined **in that** the displacement of the stability boundary of the control circuit for the axial magnetic support of the rotor (1) is determined and from this displacement the liquid damping ($D_{FL}$) is then determined.

**20.** A method in accordance with any one of the claims 4 to 12 and in accordance with any one of claims 13 to 19, **characterized in that** for the determination of the through-flow (Q) from the previously determined pressure (p) and/or for the determination of the through-flow (Q) from the drive torque (M) and the associated rotational speed (ω) the determined viscosity (η) is considered.

## Revendications

**1.** Procédé pour déterminer la pression (p) et/ou le débit (Q) d'un liquide dans ou à travers une pompe centrifuge ou une pompe diagonale ou une pompe axiale, qui comporte au moins un palier magnétique pour le montage axial du rotor (1) de la pompe, sachant que ledit palier magnétique axial du rotor de la pompe est réalisé sous forme de palier actif ou passif, et que des forces axiales s'exercent sur le rotor (1) en cours de service sous l'effet de la pression à l'intérieur de la pompe, **caractérisé en ce que** les forces axiales exercées sur le rotor (1) sont calculées pour déterminer la pression (p) et/ou le débit (Q), et **en ce que** pour le calcul des forces axiales exercées sur le rotor (1), il est tenu compte du flux du palier nécessaire pour le montage axial du rotor (1) dans le cas d'une conception active du palier magnétique axial, et il est tenu compte de la déviation axiale (z, Δz) du rotor (1) dans le cas d'une conception passive du palier magnétique axial.

**2.** Procédé selon la revendication 1, **caractérisé en ce que** différents flux du palier, nécessaires pour le montage axial du rotor (1), ou différentes déviations axiales (z, Δz) du rotor (1), chacune en présence de différentes vitesses de rotation (ω), ainsi que les pressions qui en résultent dans chaque cas sont enregistrés dans une table à consulter électronique, de telle sorte qu'en cours de service de la pompe, le flux du palier respectif ou la déviation axiale (z, Δz) respective du rotor (1), ainsi que sa vitesse de rotation (ω) sont calculés, et la pression (p) qui en résulte est alors déterminée à l'appui de ladite table à consulter.

**3.** Procédé selon la revendication 1, **caractérisé en ce que** la pression (p) à l'intérieur de la pompe est déterminée au moyen d'un polynôme à partir du flux du palier respectif, nécessaire pour le montage axial du rotor, ou à partir de la déviation axiale (z, Δz) respective du rotor (1), d'une part, ainsi qu'à partir de la vitesse de rotation (ω) respective, d'autre part.

**4.** Procédé selon la revendication 2 ou 3, **caractérisé en ce qu'**à partir de la pression (p) déjà déterminée et de la vitesse de rotation (ω) correspondante, est alors déterminé le débit (Q) à travers la pompe par l'intermédiaire des courbes caractéristiques du débit en fonction de la pression de la pompe.

5. Procédé selon la revendication 4, **caractérisé en ce que** les courbes caractéristiques du débit en fonction de la pression de la pompe sont enregistrées dans une table à consulter électronique, de telle sorte qu'en cours de service de la pompe, le débit (Q) à travers la pompe est alors déterminé au moyen de ladite table à consulter à partir de la pression (p) déjà déterminée.

6. Procédé selon la revendication 4, **caractérisé en ce que** les courbes caractéristiques du débit en fonction de la pression sont calculées par approximation par un polynôme, de telle sorte qu'en cours de service de la pompe, le débit (Q) à travers la pompe est alors déterminé au moyen de ce polynôme à partir de la pression (p) déjà déterminée.

7. Procédé selon l'une quelconque des revendications 1 à 6, **caractérisé en ce que** l'on utilise une pompe avec des capteurs (S1, S2, S3, S4) pour déterminer le sens du champ magnétique dans l'entrefer entre le rotor (1) et le stator (2), et **en ce que** lesdits capteurs (S1, S2, S3, S4) sont également utilisés pour déterminer la déviation axiale (z, $\Delta z$) du rotor (1).

8. Procédé selon la revendication 7, **caractérisé en ce que** le débit (Q) à travers la pompe est déterminé en calculant le couple de rotation (M) actionnant le rotor (1) et la vitesse de rotation (ω) du rotor (1), sachant que, d'une part, le flux d'actionnement magnétique ($\phi_M$) dans l'entrefer entre le rotor (1) et le stator (2), ainsi que le courant d'actionnement ($I_A$) pour le rotor (1) sont déterminés pour le calcul du couple de rotation (M), et sachant que, d'autre part, la déviation axiale (z, $\Delta z$) du rotor (1) est calculée et le couple de rotation (M) actionnant le rotor est alors calculé à partir du courant d'actionnement ($I_A$) et de la déviation axiale (z, $\Delta z$) du rotor (1), de telle sorte que le débit (Q) à travers la pompe est finalement déterminé à partir du couple de rotation (M) ainsi calculé et de la vitesse de rotation (ω) calculée.

9. Procédé selon la revendication 8, **caractérisé en ce que** différents courants d'actionnement ($I_A$) en présence de différentes déviations axiales du rotor, ainsi que le couple de rotation (M) qui en résulte dans chaque cas sont enregistrés dans une table à consulter électronique, de telle sorte qu'en cours de service de la pompe, le couple de rotation (M) est d'abord calculé au moyen de ladite table à consulter à partir de la déviation axiale (z, $\Delta z$) respective du rotor (1) et du courant d'actionnement ($I_A$) respectif, et, ensuite, le débit (Q) à travers la pompe est alors déterminé à partir du couple de rotation (M) ainsi calculé et à partir de la vitesse de rotation (ω) calculée.

10. Procédé selon la revendication 8 ou 9, **caractérisé en ce que** la pression (p) à l'intérieur de la pompe est déterminée à partir du débit (Q) déjà déterminé et à partir de la vitesse de rotation (ω) correspondante par l'intermédiaire des courbes caractéristiques du débit en fonction de la pression (p) de la pompe.

11. Procédé selon la revendication 10, **caractérisé en ce que** les courbes caractéristiques du débit en fonction de la pression de la pompe sont enregistrées dans une table à consulter électronique, de telle sorte qu'en cours de service de la pompe, la pression (p) à l'intérieur de la pompe peut alors être déterminée au moyen de ladite table à consulter à partir du débit (Q) déjà déterminé.

12. Procédé selon la revendication 10, **caractérisé en ce que** les courbes caractéristiques du débit en fonction de la pression sont calculées par approximation par un polynôme, de telle sorte qu'en cours de service de la pompe, la pression (p) à l'intérieur de la pompe est alors déterminée au moyen de ce polynôme à partir du débit (Q) déjà déterminé.

13. Procédé selon l'une quelconque des revendications 4 à 12, **caractérisé en ce que** la viscosité (η) du liquide à l'intérieur de la pompe est calculée pour déterminer le débit (Q) à partir de la pression (p) déterminée au préalable ou pour déterminer le débit (Q) à partir du couple de rotation (M) et de la vitesse de rotation (ω).

14. Procédé selon la revendication 13, **caractérisé en ce que** pour calculer la viscosité (η) du liquide, la pression (p), déterminée au moyen des forces axiales exercées sur le rotor (1), est comparée à la pression (p) qui est obtenue à partir de la détermination au moyen du couple de rotation (M) calculé et de la vitesse de rotation (ω) calculée, et **en ce que** la viscosité (η) est alors déterminée à partir de la différence de ces deux pressions et de la vitesse de rotation correspondante.

15. Procédé selon la revendication 13, **caractérisé en ce que** l'amortissement hydraulique ($D_{FL}$) exercé sur le rotor (1) est calculé pour déterminer la viscosité ($\eta$) du liquide, et la viscosité ($\eta$) est alors déterminée à partir de l'amortissement hydraulique ($D_{FL}$) calculée.

16. Procédé selon la revendication 15, **caractérisé en ce que** l'amortissement hydraulique ($D_{FL}$) est calculé au moyen de la déviation des propres fréquences du rotor (1).

17. Procédé selon la revendication 15, **caractérisé en ce que** l'amortissement hydraulique ($D_{FL}$) est calculé lorsque le rotor (1) fonctionne à sa propre fréquence, et la déviation du rotor qui en résulte est calculée, l'amortissement hydraulique ($D_{FL}$) étant alors déterminé au moyen de cette dernière.

18. Procédé selon la revendication 15, **caractérisé en ce que** l'amortissement hydraulique ($D_{FL}$) est calculé en faisant varier la vitesse de rotation ($\omega$) du rotor (1) avec des fréquences différentes, et la variation des forces axiales du palier ou de la déviation axiale, laquelle se produit à chaque fréquence de variation de la vitesse de rotation, est calculée, et **en ce que** l'amortissement hydraulique ($D_{FL}$) est alors calculé à partir de la variation des forces axiales du palier ou de la déviation axiale du rotor (1) en présence de différentes fréquences de variation de la vitesse de rotation.

19. Procédé selon la revendication 15, **caractérisé en ce que** l'amortissement hydraulique ($D_{FL}$) est calculé en calculant le décalage du bord de stabilité du circuit de réglage pour le palier magnétique axial du rotor (1), et l'amortissement hydraulique ($D_{FL}$) est alors calculé à partir de ce décalage.

20. Procédé selon l'une quelconque des revendications 4 à 12 et selon l'une quelconque des revendications 13 à 19, **caractérisé en ce que** la viscosité ($\eta$) calculée est prise en compte pour la détermination du débit (Q) à partir de la pression (p) déterminée au préalable ou pour la détermination du débit (Q) à partir du couple de rotation (M) et de la vitesse de rotation ($\omega$).

## Fig.1

P

ω=5000 U/min

ω=4600 U/min

ω=4200 U/min

ω=3800 U/min

ω=3400 U/min

Z

## Fig.2

P

ω=5000 U/min

ω=4600 U/min

ω=4200 U/min

ω=3800 U/min

ω=3400 U/min

Q

Fig.3

Fig.4

Fig.5

## Fig.6

## Fig.7

Fig.8

Fig.9

## Fig.10

## Fig.11

# Fig.12

# Fig.13

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19613388 A
- US 5735357 A
- US US5613831 A
- US US4779614 A
- US US4341111 A
- US 4781525 A